# EUROPEAN PATENT APPLICATION

(11) **EP 3 560 361 A1**
(43) Date of publication of application: **30.10.2019**
(21) Application number: 17885246.3
(22) Date of filing: 02.06.2017
(51) Int. Cl.: A24F 47/00

(54) **UPPER COVER ASSEMBLY, ATOMIZATION DEVICE AND ELECTRONIC CIGARETTE**

(30) Priority: 21.12.2016 CN 201621406359 U
(71) Applicant: Changzhou Patent Electronic Technology Co., Ltd, Jiangsu Province 213001 (CN)
(72) Inventor: QIU, Weihua, Xinbei District, Changzhou City Jiangsu Province 213001 (CN)
(74) Representative: Haseltine Lake Kempner LLP
(86) International application number: PCT/CN2017/087007
(87) International publication number: WO 2018/113195

(57) **Abstract**

Provided is an upper cover assembly (40), comprising an upper cover (41) and a liquid filling cover (43) movable between a locked position and an unlocked position relative to the upper cover (41). The upper cover (41) is provided with a liquid filling port (412). When the liquid filling cover (43) is in the locked position relative to the upper cover (41), the liquid filling cover (43) covers the upper cover (41) and is unable to be flipped relative to the upper cover (41), and the liquid filling port (412) is closed; and when the liquid filling cover (43) is translated, by an external force, from the locked position to the unlocked position relative to the upper cover (41), the liquid filling cover (43) is able to be flipped and opened relative to the upper cover (41), and the liquid filling port (412) is exposed. When the liquid filling cover (43) of the electronic cigarette is in the locked position, the liquid filling cover is in communication with a vapour outlet (410), the electronic cigarette can operate vaping normally; and when the liquid filling cover (43) is translated from the locked position to the unlocked position and then is flipped and opened, the liquid filling port (412) is exposed, and the electronic cigarette can operate liquid filling. An atomization device (200) with the upper cover assembly (40) and an electronic cigarette are further provided.

## Description

### TECHNICAL FIELD

The present invention relates to the technical filed of electronic cigarettes, and in particular to an upper cover assembly, an atomizing device and an electronic cigarette.

### BACKGROUND

The atomizing device of the traditional electronic cigarette needs to unscrew the upper cover or the bottom seat when the liquid is required to be filled, which is inconvenient to operate, and the process of disassembling the upper cover or the bottom seat is cumbersome. Further, when the upper cover of the atomizing device is unscrewed for liquid filling, the pressure within the atomizing device is easily changed to cause liquid leakage, causing great inconvenience to the user. Meanwhile, the children are also easily accessible to the e-cigarette liquid, there are security risks.

### SUMMARY

In view of above, the present invention provides an upper cover assembly that facilitates liquid filling operation and does not cause pressure changes in the chamber of the atomizing device when the liquid filling cover is opened.

It is further to provide an atomizing device having the upper cover assembly.

It is more further to provide an electronic cigarette having the atomizing device.

The technical solution adopted by the present invention to solve the problem is as follows.

An upper cover assembly includes an upper cover and a liquid filling cover movable relative to the upper cover between a locked position and an unlocked position. The upper cover is provided with a liquid filling hole. When the liquid filling cover is in the locked position relative to the upper cover, the liquid filling cover covers the upper cover and is unable to be flipped relative to the upper cover, and the liquid filling hole is closed. When the liquid filling cover is moved, by an external force, from the locked position to the unlocked position relative to the upper cover, the liquid filling cover is able to be flipped and opened relative to the upper cover, and the liquid filling hole is exposed.

Further, the upper cover is provided with a sliding groove for the liquid filling cover to move between the locked position and the unlocked position. The liquid filling cover is provided with a rotating shaft. The rotating shaft is movable along the sliding groove. The liquid filling cover is able to be flipped relative to the upper cover around the rotating shaft.

Further, the upper cover includes a bottom cover and a gland disposed on the bottom cover. The liquid filling hole is provided in the bottom cover. An edge of one side of the gland is recessed inwardly to form a notch having an opening at one end. The liquid filling hole is received in the notch and exposed through the notch.

Further, the notch includes a connecting wall located at a side of the liquid filling hole and two side walls connected to opposite ends of the connecting wall and extending in the same direction. The surface of the connecting wall near the bottom cover is recessed to form a recess. The surface of each of the two side walls near the bottom cover is recessed to form the sliding groove at the position near the connecting wall. The sliding groove is in communication with the notch and the recess. One end of the liquid filling cover is received and confined in the recess in the locked position. When the end of the liquid filling cover is moved out of the recess along the sliding groove from the locked position to the unlocked position, the liquid filling cover is able to be flipped.

Further, the liquid filling cover includes a cover body. The cover body is able to close the liquid filling hole when the liquid filling cover is in the locked position. The rotating shaft is disposed at two opposite sides of one end of the cover body.

Further, the liquid filling cover includes a covering portion disposed on the other end of the cover body opposite to the rotating shaft. The covering portion extends to both sides of the cover body and has a width greater than the width of the notch in the circumferential direction of the gland. The bottom of the gland on both sides of the notch is provided with a locking groove in communication with the notch. The width of the locking groove is larger than the width of the notch in the circumferential direction of the gland. When the liquid filling cover is in the locked position, the covering portion is received in the locking groove and resists the groove wall of the locking groove. When the liquid filling cover is in the unlocked position, the covering portion moves out of the locking groove, the liquid filling cover is able to be flipped relative to the upper cover.

Further, the upper cover is further provided with a smoke outlet. The smoke outlet and the liquid filling hole are sequentially arranged in the upper cover along the direction from the locked position to the unlocked position. The liquid filling cover is provided with a through hole. The through hole is in communication with the smoke outlet when the liquid filling cover is in the locked position relative to the upper cover.

Further, the upper cover includes a first connecting portion for restricting the liquid filling cover from being flipped when the liquid filling cover is in the locked position, and the liquid filling cover is provided with a second connecting portion for being locked or unlocked with the first connecting portion.

Further, the upper cover includes a bottom cover and a stopping member. The bottom cover is provided with an engaging groove. The stopping member is engaged in the engaging groove. Two opposite side walls along the length direction of the stopping member are provided with the sliding groove.

Further, the liquid filling cover includes a locking member. The locking member closes the liquid filling hole when the liquid filling cover is in the locked position. The rotating shaft is provided on the locking member. The rotating shaft is movable along the sliding groove to be locked with the stopping member.

Further, the surface of the stopping member away from the liquid filling cover is defined with a clamping groove. One end of the stopping member opposite to the clamping groove is provided with a limiting groove. One end of the locking member is provided with a claw slidably received in the clamping groove, and the other end of the locking member opposite to the claw is provided with a limiting portion slidably received in the limiting groove. The rotating shaft is provided on the limiting portion.

Further, a surface of the stopping member facing the liquid filling cover is provided with an inserting opening in communication with the clamping groove. The claw is movable in the clamping groove after passing through the inserting opening. The claw is provided with a latching groove. A projection corresponding to the latching groove is provided in the clamping groove. When the liquid filling cover is in the locked position, the projection is snapped in the latching groove, and the circumference of the inserting opening abuts against the claw.

Further, the first connecting portion and the second connecting portion are each provided with a magnetic member. When the liquid filling cover is in the locked position, the first connecting portion is magnetically connected to the second connecting portion.

Further, the upper cover further includes an elastic member. The elastic member includes a clamping portion clamped between the bottom cover and the stopping member and a resisting portion connected to the clamping portion. The resisting portion is connected to one end of the clamping portion away from the inserting opening. One end of the resisting portion away from the clamping portion is inclined in a direction away from the bottom wall of the engaging groove. When the liquid filling cover moves between the locked position and the unlocked position, the resisting portion always abuts against the limiting portion.

Further, the upper cover further includes an upper cover casing sleeved on the bottom cover. The liquid filling cover further includes a cover body connected to one side of the locking member away from the upper cover. The upper cover casing is provided with a smoke outlet. The cover body is provided with a through hole. The through hole is in communication with the smoke outlet when the liquid filling cover is in the locked position relative to the upper cover.

An atomizing device includes any of the above upper cover assemblies.

Further, the atomizing device further includes a base assembly, a liquid storage tube and an atomizing assembly. The liquid storage tube is sleeved on the base assembly. A liquid storage chamber is surrounded cooperatively by the liquid storage tube and the base assembly. The atomizing assembly is received in the liquid storage chamber. The upper cover assembly is disposed at one end of the liquid storage tube opposite to the base assembly and is in communication with the liquid storage chamber through the liquid filling hole.

An electronic cigarette includes any of the above atomizing devices.

The beneficial effects of the present invention are:

For the electronic cigarette of the present invention, when the liquid filling cover is in the locked position, the liquid filling hole is closed. When the remaining amount of the e-cigarette liquid in the electronic cigarette is insufficient, the liquid filling cover can be moved from the locked position to the unlocked position, and then flipped to expose the liquid filling hole to realize the liquid filling. During the operation, the liquid filling cover can be flipped only after being pushed out relative to the upper cover, and it cannot be directly flipped. Flipping or pushing individually cannot expose the liquid filling hole, the liquid filling hole can be opened only by a two-step operation with a pushing and a flipping, so it is childproof.

### BRIEF DESCRIPTION OF THE DRAWINGS

The present invention will now be further described with reference to the accompanying drawings and embodiments.
FIG.1 is a schematic view showing the structure of an atomizing device according to a first embodiment of the present invention (the liquid filling cover is in the flipped state);
FIG. 2 is an exploded view of the atomizing device of FIG. 1;
FIG. 3 is a schematic view showing the structure of the base assembly and the liquid storage tube of the atomizing device of FIG. 1;
FIG. 4 is a schematic view showing the structure of the atomizing assembly of the atomizing device of FIG. 1;
FIG. 5 is a schematic view showing the structure of the upper cover assembly of the atomizing device of FIG. 1;
FIG. 6 is a cross-sectional view of the upper cover assembly of the atomizing device of FIG. 5 after being assembled;
FIG. 7 is a cross-sectional view of the atomizing device of FIG. 1;
FIG. 8 is a schematic view showing the structure of an atomizing device according to a second embodiment of the present invention (the liquid filling cover is in the flipped state);
FIG. 9 is an exploded view of the upper cover assembly of the atomizing device of FIG. 8;
FIG. 10 is an exploded view of the upper cover assembly of the atomizing device of FIG. 9 from another viewing angle;
FIG. 11 is a schematic view showing the structure of the bottom cover of the upper cover assembly of the atomizing device of FIG. 8;
FIG. 12 is a cross-sectional view of the upper cover assembly of the atomizing device of FIG. 8 after being assembled (the liquid filling cover is in the locked position);
FIG. 13 is a cross-sectional view showing another state of the upper cover assembly of the atomizing device of FIG. 8 after being assembled (the liquid filling cover is in the unlocked position);
FIG. 14 is a cross-sectional view of the atomizing device of FIG. 8.

The following table lists the various components and reference numerals of the figures.

| | | |
|---|---|---|
| atomizing device 100, 200 | base assembly 10 | inner chamber 110 |
| bottom seat 11 | air inlet 112 | air adjusting ring 13 |
| air adjusting groove 130 | liquid storage tube 20 | liquid storage chamber 21 |
| atomizing assembly 30 | atomizing chamber 310 | atomizing tube 31 |
| electrode contact assembly 33 | liquid inlet hole 312 | upper cover assembly 40 |
| smoke outlet 410 | liquid filling hole 412 | through hole 430 |
| bottom cover 414 | connection fixing post 4141, 4111 | sealing ring 418 |
| sealing groove 4143 | sealing pad 45 | gland 416 |
| notch 4161 | connecting wall 4162 | locking groove 4163 |
| side wall 4164 | recess 4166 | sliding groove 4165, 4156 |
| liquid filling cover 43 | rotating shaft 431 | cover body 433 |
| covering portion 432 | pin 417 | upper cover 41 |
| upper cover casing 411 | hole 4142 | engaging groove 4144 |
| first convex 4145 | second convex 4146 | slot 4147 |
| pivotal hole 4347 | stopping member 415 | clamping groove 4151 |
| inserting hole 4153 | inserting opening 4154 | limiting groove 4155 |
| limiting portion 4346 | fastening member 420 | projection 4181 |
| elastic member 419 | clamping portion 4191 | resisting portion 4192 |
| locking member 434 | plug portion 4331 | alignment hole 4341 |
| first connecting member 4342 | claw 4343 | latching groove 4344 |
| second connecting member 4345 | mouthpiece 50 | first connecting portion 422 |
| second connecting portion 421 | | |

### DETAILED DESCRIPTION OF PREFERRED EMBODIMENTS

In order to facilitate the understanding of the present invention, the present invention will be described more fully below with reference to the accompanying drawings. Preferred embodiments of the invention are shown in the drawings. However, the invention may be embodied in many different forms and is not limited to the embodiments described herein. Rather, these embodiments are provided so that the understanding of the disclosure of the invention may be more thorough.

It should be noted that when an element is referred to as being "fixed" to another element, it may be directly on the another element or intervening elements may be present. When an element is referred to be "connected" to another element, it may be connected to the another element directly or through intervening elements.

All technical terms used herein have the same meaning as commonly understood by one of ordinary skill in the art to the present invention. The terminology used in the present invention is for the purpose of describing particular embodiments and is not intended to limit the invention. The term "and/or" used herein includes any and all combinations of one or more of the associated listed items.

### First embodiment

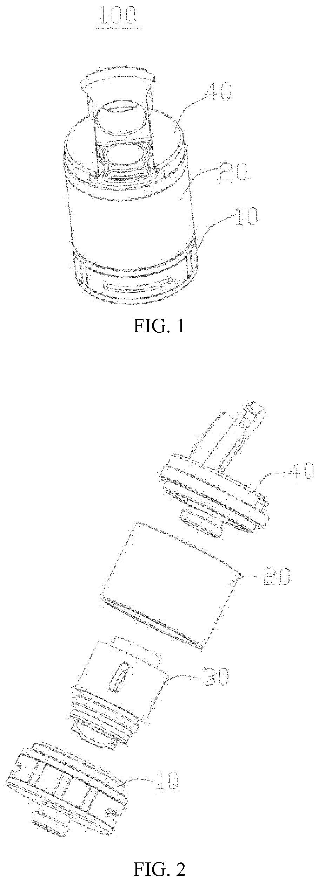

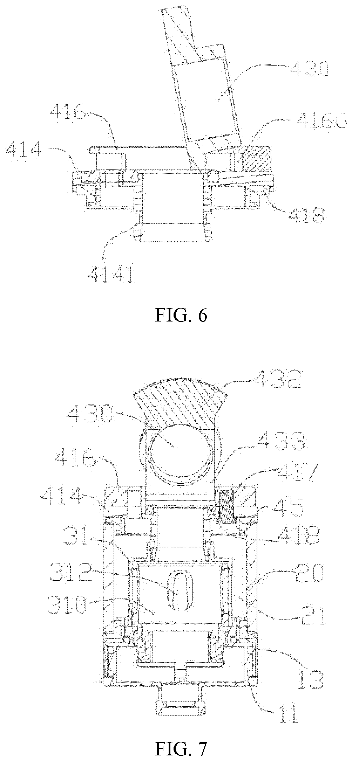

Referring to FIG. 1 and FIG. 2, in an embodiment of the present invention, an electronic cigarette (not shown) includes an atomizing device 100 and a battery device (not shown) for electrically driving the atomizing device 100. The atomizing device 100 includes a base assembly 10, a liquid storage tube 20, an atomizing assembly 30 provided with an atomizing chamber 310 (FIG. 7), an upper cover assembly 40 and a mouthpiece (not shown). The liquid storage tube 20 is sleeved on the base assembly 10. A liquid storage chamber 21 (FIG. 7) for storing e-cigarette liquid is surrounded cooperatively by the liquid storage tube 20 and the base assembly 10. The atomizing assembly 30 is received in the liquid storage chamber 21 for atomizing the e-cigarette liquid. The upper cover assembly 40 is disposed at one end of the liquid storage tube 20 opposite to the base assembly 10, and the upper cover assembly 40 is selectively in communication with the atomizing chamber 310 and the liquid storage chamber 21. When the upper cover assembly 40 is communicated with the atomizing chamber 310, the user is convenient to inhale the smoke formed by atomization. When the upper cover assembly 40 is communicated with the liquid storage chamber 21, the air pressure in the liquid storage chamber 21 can be kept stable, the liquid leakage is prevented, and it is convenient for the user to fill the e-cigarette liquid. The mouthpiece is detachably disposed on the upper cover assembly 40 for the user to inhale the smoke formed after atomization.

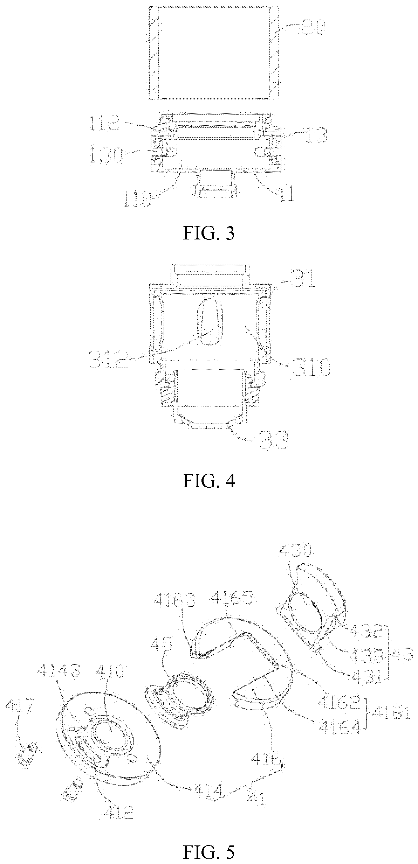

Referring to FIG. 3, specifically, the base assembly 10 is substantially hollow cylindrical. The base assembly 10 includes a bottom seat 11 provided with an inner chamber 110 and an air adjusting ring 13. The bottom seat 11 has a substantially hollow cylindrical shape, and an air inlet 112 communicating with the inner chamber 110 is defined in a circumferential wall of the bottom seat 11. The air distribution ring 13 is circumferentially rotatably sleeved on the outer circumference of the bottom seat 11 and has an air adjusting groove 130 communicating with the outside environment. The air adjusting groove 130 can be in communication with or staggered from the air inlet 112. When the air adjusting ring 13 is rotated, the communication area between the air adjusting groove 130 and the air inlet 112 can be adjusted, so that the user can adjust the amount of the air intake as needed.

The liquid storage tube 20 is a hollow tube with two ends thereof being opened. Optionally, the liquid storage tube 20 is a glass tube or a stainless steel tube, which is sleeved outside the bottom seat 11, and the liquid storage tube 20 and the bottom seat 11 together define a liquid storage chamber 21 for storing the e-cigarette liquid.

Referring to FIG. 4, the atomizing assembly 30 is received in the liquid storage chamber 21. The atomizing assembly 30 includes an atomizing tube 31, a heating member (not shown), a liquid guiding member (not shown) and an electrode contact assembly 33. The atomizing tube 31 has openings at the upper and lower ends, and an atomizing chamber 310 is formed in the atomizing tube 31. The atomizing tube 31 is provided with a liquid inlet hole 312 communicating the liquid storage chamber 21 with the atomizing chamber 310. The heating member and the liquid guiding member are disposed in the atomizing tube 31 for absorbing the e-cigarette liquid in the liquid storage chamber 21 and heating and atomizing the e-cigarette liquid using the electric power from the battery device. The heating member may be a heating wire, the liquid guiding member may be a liquid guiding cotton. The arrangement can be that the liquid guiding cotton is wrapped around the heating wire, or the heating wire is wound around the liquid guiding member. The electrode contact assembly 33 is disposed at the bottom of the atomizing tube 31, one end is electrically connected to the heating member, and the other end is electrically connected to the battery device to provide electrical power to the atomizing assembly 30.

The external air enters the inner chamber 110 of the bottom seat 11 from the air adjusting groove 130 of the air adjusting ring 13 and the air inlet 112 of the bottom seat 11, and then enters the atomizing chamber 310 from the lower end of the atomizing tube 31. The e-cigarette liquid in the atomizing chamber 310 is atomized to form smoke, the external air and the smoke pass through the top of the atomizing tube 31 and the upper cover assembly 40, and then flow into the mouthpiece for the user to inhale.

Referring to FIG. 5 and FIG. 6, the upper cover assembly 40 includes an upper cover 41 and a liquid filling cover 43 that can move relative to the upper cover 41 between a locked position and an unlocked position. The upper cover 41 is sequentially provided with a smoke outlet 410 and a liquid filling hole 412 along the moving path of the liquid filling cover 43 from the locked position to the unlocked position. The liquid filling cover 43 is provided with a through hole 430. When the liquid filling cover 43 is in the locked position relative to the upper cover 41, the liquid filling cover 43 covers the upper cover 41 and cannot be flipped relative to the upper cover 41, the through hole 430 is in communication with the smoke outlet 410, and the liquid filling hole 412 is closed. When the liquid filling cover 43 is moved, by an external force, from the locked position to the unlocked position relative to the upper cover 41, the liquid filling cover 43 can be flipped and opened relative to the upper cover 41, and the liquid filling hole 412 is exposed.

In this way, when the electronic cigarette is in use, the through hole 430 of the liquid filling cover 43 is located at the locked position and is in communication with the smoke outlet 410, so that the smoke formed by atomization in the atomizing chamber 310 flows out through the through hole 430 for the user to inhale. At this moment, the liquid filling hole 412 is closed, and the liquid filling operation cannot be performed. When the liquid filling operation is required, the through hole 430 is firstly moved from the locked position to the unlocked position by pushing it horizontally, and then flipped and opened relative to the upper cover 41, such that the liquid filling hole 412 is exposed. The air pressure in the liquid storage chamber 21 is stabilized by pre-relieving, so as to avoid the liquid leakage caused by the change of the air pressure in the liquid storage chamber 21 due to the sudden opening of the liquid filling cover 43, and the liquid filling operation can be performed at this time.

Specifically, the upper cover 41 includes a bottom cover 414 and a gland 416 disposed above the bottom cover 414. The smoke outlet 410 and the liquid filling hole 412 are sequentially provided in the bottom cover 414 along the moving path of the liquid filling cover 43 from the locked position to the unlocked position. The bottom cover 414 is disposed at one end of the liquid storage tube 20 away from the base assembly 10, and a surface of the bottom cover 414 away from the gland 416 is protruded to form a connection fixing post 4141 having a hollow structure. The connection fixing post 4141 is inserted into one end of the atomizing tube 31 away from the bottom seat 11 for fixing the upper cover assembly 40 and the atomizing assembly 30 to allow the smoke in the atomizing chamber 310 to flow out. In addition, the upper cover 41 further includes a sealing ring 418 disposed between the bottom cover 414 and the liquid storage tube 20 for preventing leakage of the e-cigarette liquid.

An edge of one side of the gland 416 is recessed inwardly to form a notch 4161 that is open at one end and has a U-shaped structure, both the smoke outlet 410 and the liquid filling hole 412 are received in and exposed through the notch 4161. Specifically, the notch 4161 includes a connecting wall 4162 located at a side of the smoke outlet 410 away from the liquid filling hole 412 and two side walls 4164 connected to opposite ends of the connecting wall 4162 and extending in the same direction. The surface of the connecting wall 4162 near the bottom cover 414 is recessed to form a recess 4166 in a direction away from the smoke outlet 410, so that a receiving gap having a certain height is formed between the gland 416 and the bottom cover 414. The surface of each of the two side walls 4164 near the bottom cover 414 is recessed to form a sliding groove 4165 at the position near the connecting wall 4162, and the sliding groove 4165 is in communication with the notch 4161 and the recess 4166. The two sliding grooves 4165 are in communication with the recess 4166 to form a U-shaped groove that surrounds the notch 4161 and has the same opening direction as the notch 4161.

One end of the liquid filling cover 43 is received and confined in the recess 4166 in the locked position. When the end of the liquid filling cover 43 is moved out of the recess 4166 along the sliding groove 4165 from the locked position to the unlocked position, the liquid filling cover 43 can be flipped. Specifically, the liquid filling cover 43 includes a cover body 433 and a rotating shaft 431 protruding from opposite sides of one end of the cover body 433. The cover body 433 has a substantially rectangular plate shape, and its shape matches the shape of the notch 4161. The through hole 430 extends through the cover body 433, and the hole wall of the through hole 430 protrudes out of the surface of the cover body 433 away from the bottom cover 414. When the liquid filling cover 43 is in the locked position, the rotating shaft 431 is locked in the recess 4166, the hole wall of the through hole 430 abuts against the recess wall of the recess 4166, to prevent the gland 416 from being flipped around the rotating shaft 431. When the liquid filling cover 43 is moved from the locked position to the unlocked position by an external force, referring to FIG. 6, the rotating shaft 431 moves out of the recess 4166 along the sliding grooves 4165, so that the hole wall of the through hole 430 and the recess wall of the recess 4166 forms a rotational space for the cover body 433 to be capable of flipping around the rotating shaft 431 relative to the upper cover 43. At this time, the liquid filling cover 43 can be flipped and opened relative to the upper cover 41, so that the liquid filling hole 412 is exposed and the user can perform the liquid filling operation. After the liquid filling operation is finished, the liquid filling cover 43 is caused to cover on the upper cover 41 and then pushed back to the locked position from the unlocked position. In the whole process, the cover body 433 cannot be directly flipped in the locked position, and it is necessary to perform the push to cancel the interference between the cover body 433 and the gland 416, in order to realize flipping of the cover body 433. Thus, the liquid filling cover 43 is prevented from being opened during the use of the electronic cigarette, and provides with a child protection function at the same time.

The liquid filling cover 43 includes a covering portion 432 disposed on the other end of the cover body 433 opposite to the rotating shaft 431. The covering portion 432 extends outwards from both sides of the cover body 433, so that the covering portion 432 has a width greater than the width of the notch 4161 in the circumferential direction of the gland 416. In the specific embodiment, the covering portion 432 has a fan shape that extends toward the both sides of the cover body 433. At the same time, the bottom of the gland 416 on both sides of the notch 4161 is provided with a locking groove 4163 in communication with the notch 4161, and the width of the locking groove 4163 in the circumferential direction of the gland 416 is larger than the width of the notch 4161. That is, the locking groove 4163 and the notch 4161 form a stepped groove with a wider upper part and a narrower lower part. Thus, when the liquid filling cover 43 is pushed into the locked position relative to the upper cover 41, the covering portion 432 is received in the locking groove 4163 and resists the groove wall of the locking groove 4163, to prevent the liquid filling cover 43 from being pushed in continuously. When the liquid filling cover 43 is pushed out to the unlocked position relative to the upper cover 41, the covering portion 432 moves out of the locking groove 4163 through the opening of the locking groove 4163, so that the liquid filling cover 43 can be flipped relative to the upper cover 41.

In the specific embodiment, the liquid filling hole 412 is a circular arc shape surrounding the partial outer circumference of the smoke outlet 410. The size of the liquid filling hole 412 is increased to facilitate the liquid filling operation while being matched with the shape of the sector-shaped covering portion 432.

Further, in order to ensure the tightness between the gland 416 and the liquid filling cover 43, a sealing groove 4143 is defined in the bottom cover 414 around the smoke outlet 410 and the liquid filling hole 412. The upper cover assembly 40 includes a sealing pad 45, the sealing pad 45 is received in the sealing groove 4143 and sealed around the outer periphery of the smoke outlet 410 and the liquid filling hole 412.

Referring also to FIG. 7, in the specific embodiment, the upper cover 41 includes a pin 417, the pin 417 passes through the bottom cover 414 from below and is fixed on the gland 416, to thus fix the bottom cover 414 and the gland 416. It is to be understood that in other embodiments, the fixing manner of the gland 416 and the bottom cover 414 can be varied as needed, or the two can be integrally formed, which is not limited herein.

When the liquid filling cover 43 of the electronic cigarette of the present invention is in the locked position, the liquid filling cover 43 covers the upper cover 41 and cannot be flipped relative to the upper cover 41, the smoke outlet 410 is in communication with the through hole 430, and the liquid filling hole 412 is closed. At this time, the smoking operation can be performed normally and the liquid filling operation cannot be performed. When the remaining amount of the e-cigarette liquid in the electronic cigarette is insufficient, the liquid filling cover 43 is moved from the locked position to the unlocked position to completely expose the liquid filling hole 412 by flipping the liquid filling cover 43 for liquid filling. During the operation, the liquid filling cover 43 can be flipped only after being pushed out to the unlocked position relative to the upper cover 41, and it cannot be directly flipped. If the liquid filling cover 43 is directly pushed, the covering portion 432 still covers the liquid filling hole 412, that is, flipping or pushing individually cannot expose the liquid filling hole, the liquid filling hole can be opened only by a two-step operation with a pushing and a flipping, so it is childproof.

### Second embodiment

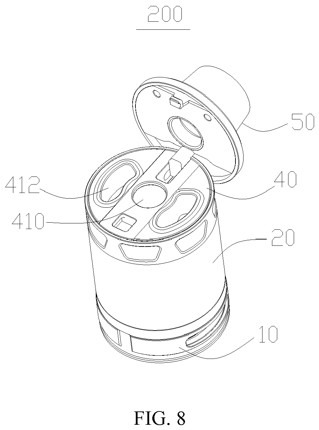

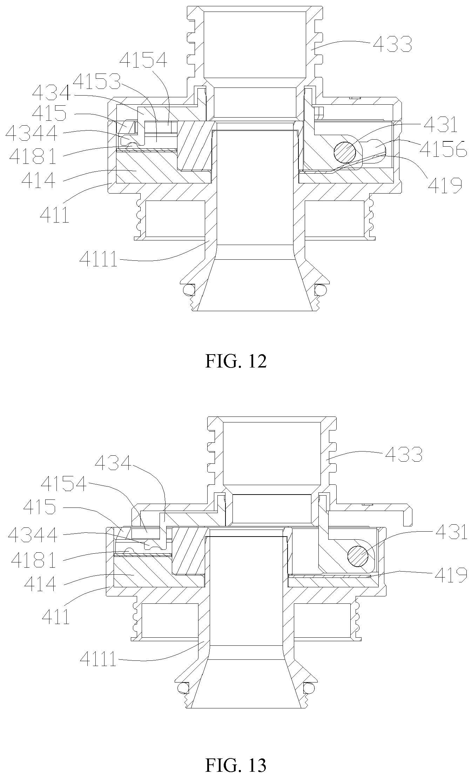

Referring to both FIG. 8 and FIG. 12, the second embodiment of the present invention provides an electronic cigarette (not shown) that includes an atomizing device 200 and a battery device (not shown) for electrically driving the atomizing device 200. The atomizing device 200 includes a base assembly 10, a liquid storage tube 20, an atomizing assembly 30, an upper cover assembly 40 and a mouthpiece 50. The difference between the atomizing device 200 and the atomizing device 100 of the first embodiment is that the structure of the upper cover assembly 40 is different, other structures are the same as those in the first embodiment, and are not described herein again.

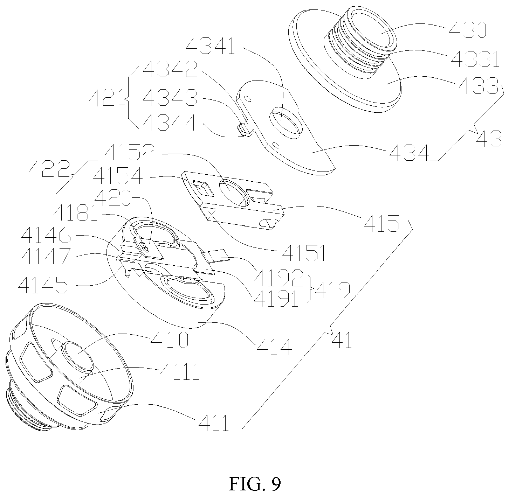

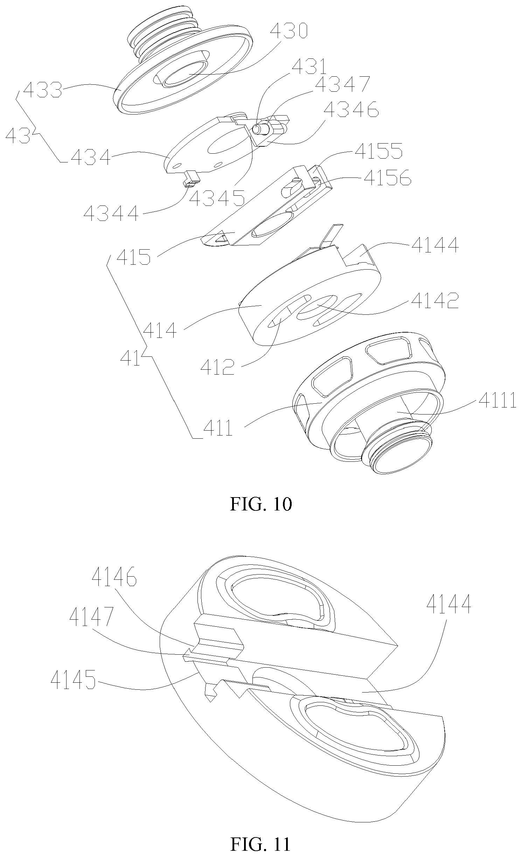

Specifically, referring also to FIG. 9 and FIG. 10, the upper cover assembly 40 includes an upper cover 41 and a liquid filling cover 43 that can move relative to the upper cover 41 between a locked position and an unlocked position. The upper cover 41 is provided with a smoke outlet 410 in communication with the atomizing chamber 310 and a liquid filling hole 412 in communication with the liquid storage chamber 21. A through hole 430 is provided in the liquid filling cover 43. The upper cover 41 includes a first connecting portion 422 that can restrict the liquid filling cover 43 from being flipped when the liquid filling cover 43 is in the locked position. The liquid filling cover 43 is provided with a second connecting portion 421 that can be locked or unlocked with the first connecting portion 422. When the first connecting portion 422 and the second connecting portion 421 are interlocked, the liquid filling cover 43 cannot be flipped relative to the upper cover 41, and the liquid filling hole 412 is closed at this time. When the first connecting portion 422 and the second connecting portion 421 are unlocked, the liquid filling cover 43 can be flipped relative to the upper cover 41 to open the liquid filling hole 412.

The upper cover 41 includes an upper cover casing 411, a bottom cover 414 received in the upper cover casing 411, and a stopping member 415 locked at the bottom cover 414.

The upper cover casing 411 is substantially a hollow cylindrical structure having openings at both ends, the upper cover casing 411 is disposed at one end of the liquid storage tube 20 away from the base assembly 10, and a connection fixing post 4111 extends through the surface of the upper cover casing 411 facing the base assembly 10. One end of the connection fixing post 4111 is screwed to the atomizing tube 31 for fixing the upper cover assembly 40 and the atomizing assembly 30 and allowing the smoke in the atomizing chamber 310 to flow out. One end of the connection fixing post 4111 away from the atomizing assembly 30 is received in the upper cover casing 411, the smoke outlet 410 is formed by a port of the connection fixing post 4111 away from one end of the atomizing assembly 30.

The bottom cover 414 has a generally cylindrical structure, a hole 4142 is provided at the center of the bottom cover 414 and extends through two opposite ends of the bottom cover 414. The bottom cover 414 is fixedly received in the upper cover casing 411. When the bottom cover 414 is received in the upper cover casing 411, one end of the connection fixing post 4111 received in the upper cover casing 411 passes through the hole 4142. A surface of the bottom cover 414 facing the liquid filling cover 43 is recessed to form an engaging groove 4144 in a radial direction of the bottom cover 414. The engaging groove 4144 is configured to engage with the stopping member 415 to secure the stopping member 415. The engaging groove 4144 is substantially elongated, both ends of the engaging groove 4144 pass through the peripheral wall of the bottom cover 414. The engaging groove 4144 has a width greater than the outer diameter of the hole 4142 and is in communication with the hole 4142. Referring to FIG. 11, a first convex 4145 is protruded from the bottom of one end of the engaging groove 4144, two opposite groove walls of the engaging groove 4144 adjacent to the first convex 4145 are provided with a second convex 4146. The side wall of the second convex 4146 is formed with a slot 4147, the slot 4147 extends through the side wall of the second convex 4146 along the length direction of the engaging groove 4144, the space between the top surface of the first convex 4145 and the top wall of the slot 4147 constitutes an insertion groove (not labeled).

In this embodiment, the number of the liquid filling hole 412 is two, the liquid filling holes 412 are disposed at opposite sides of the engaging groove 4144 of the bottom cover 414. The liquid filling hole 412 extends through the upper and lower surfaces of the bottom cover 414. It can be understood that, in other embodiments not shown, there is at least one liquid filling hole 412, the liquid filling hole 412 is defined at the top end of the upper cover 41, for example, a liquid filling hole 412 is provided and defined at one side of the engaging groove 4144.

The upper cover 41 further includes a fastening member 420. The fastening member 420 has a substantially sheet-like structure and is closely inserted into the insertion groove. When the fastening member 420 is installed, it is only necessary to insert the fastening member 420 into the insertion groove along the open end of the insertion groove. In this embodiment, the surface of the fastening member 420 facing the liquid filling cover 43 is provided with two projections 4181, and the projection 4181 is made of elastic material. When the projection 4181 is subject to an external force, deformation occurs, and when the external force is removed, the projection 4181 can be reset again.

The stopping member 415 is generally elongated. The surface of the stopping member 415 away from the liquid filling cover 43 is defined with a clamping groove 4151 corresponding to the second convex 4146. The stopping member 415 is provided with an inserting hole 4152 corresponding to the connection fixing post 4111. The inserting hole 4152 extends through the upper and lower surfaces of the stopping member 415. When the stopping member 415 is engaged in the engaging groove 4144, the connection fixing post 4111 is inserted into the inserting hole 4152, the bottom wall of the clamping groove 4151 abuts on the second convex 4146. Thus, a hollow chamber 4153 is formed between the stopping member 415 and the fastening member 420. Obviously, the hollow chamber 4153 is a portion of the clamping groove 4151. In this embodiment, the surface of the stopping member 415 facing the liquid filling cover 43 is defined with an inserting opening 4154 in communication with the hollow chamber 4153. In this embodiment, the first connecting portion 422 includes the inserting opening 4154, further includes the fastening member 420 and the projection 4181.

The stopping member 415 is provided with a limiting groove 4155 at one end away from the inserting opening 4154. The limiting groove 4155 is provided along the length direction of the stopping member 415 and extends through the upper and lower surfaces of the stopping member 415. The opposite side walls of the limiting groove 4155 along the length direction of the stopping member 415 are provided with a sliding groove 4156, and the sliding grooves 4156 are generally elongated.

In the specific embodiment, the upper cover 41 further includes an elastic member 419 located between the bottom cover 414 and the stopping member 415. The elastic member 419 includes a clamping portion 4191 and a resisting portion 4192 connected to the clamping portion 4191. The clamping portion 4191 has a substantially square structure. The resisting portion 4192 has a generally elongated strip structure. The clamping portion 4191 is clamped between the bottom cover 414 and the stopping member 415. The resisting portion 4192 is connected to one end of the clamping portion 4191 away from the inserting opening 4152, and one end of the resisting portion 4192 away from the clamping portion 4191 is inclined in a direction away from the bottom wall of the engaging groove 4144. The resisting portion 4192 has elasticity, when the resisting portion 4192 is depressed, one end of the resisting portion 4192 away from the clamping portion 4191 is driven to move downwards, and when the pressure is released, the resisting portion 4192 can reset. In this embodiment, the clamping portion 4191 and the resisting portion 4192 are an integrally formed stainless steel elastic piece, it is understood that other elastic members such as springs are also applicable.

The liquid filling cover 43 includes a cover body 433 and a locking member 434 disposed under the cover body 433.

Specifically, the cover body 433 has a substantially disk-like structure, the through hole 430 is defined at the center of the cover body 433, the hole wall of the through hole 430 extends away the upper cover 41 to form the plug portion 4331. The mouthpiece 50 is sleeved on the plug portion 4331.

The locking member 434 is generally a plate having a semi-circular structure. The locking member 434 is fixedly mounted on the surface of the cover body 433 away from the mouthpiece 50. When the liquid filling cover 43 is in the locked position, the locking member 434 can seal the liquid filling hole 412, thereby closing the liquid filling hole 412. The locking member 434 is provided with an alignment hole 4341 corresponding to the through hole 430. When the locking member 434 is connected to the cover body 433, the alignment hole 4341 is in communication with the through hole 430. In this embodiment, the locking member 434 is plugged into the cover body 433. It can be understood that in other embodiments not shown, the locking member 434 can also be connected to the cover body 433 by means of a snapping or glue connection, or the locking member 434 is integrally formed with the cover body 433, and is not limited herein.

In order to improve the sealing effect of the liquid filling hole 412 by the locking member 434, the outer periphery of the liquid filling hole 412 is provided with a sealing pad 45.

The surface of the locking member 434 away from the cover body 433 is provided with a first connecting member 4342 disposed adjacent to the inserting opening 4154 of the stopping member 415. One end of the first connecting member 4342 away from the locking member 434 extends outwardly to form a claw 4343. The surface of the claw 4343 away from the locking member 434 is recessed with a latching groove 4344 that cooperates with the projection 4181. The claw 4343 can be inserted into the hollow chamber 4153 through the inserting opening 4154. When the locking member 434 is moved, the claw 4343 moves in the hollow chamber 4153 under the driving of the locking member 434, so that the projection 4181 is snapped in the latching groove 4344. In this embodiment, the second connecting portion 421 includes the first connecting member 4342, and further includes the claw 4343, still further includes the latching groove 4344. When the liquid filling cover 43 is in the locked position, the first connecting portion 422 is locked with the second connecting portion 421. When the projection 4181 is snapped into the latching groove 4344, the circumference of the inserting opening 4154 abuts against the claw 4343, thereby preventing the locking member 43 from being flipped relative to the upper cover 41. In this embodiment, the projection 4181 is made of an elastic material, the projection 4181 is deformed to be snapped in the latching groove 4344. It can be understood that in other embodiments not shown, the claw 4343 is made of an elastic material, and the claw 4343 is deformed such that the projection 4181 can snap into the latching groove 4344. Of course, it is also understood that, in other embodiments not shown, the first connecting portion 422 and the second connecting portion 421 can each be provided with a magnetic member, such as a magnet. When the liquid filling cover 43 is in the locked position, the first connecting portion 422 is magnetically connected to the second connecting portion. Further, the second connecting portion 421 includes the claw 4343, the claw 4343 itself is a magnetic member or is provided with a magnetic member. The first connecting portion 422 includes the fastening member 420 and the projection 4181, and the projection 4181 is provided with a magnetic member, such as a magnet. When the claw 4343 is moved into position, the magnetic member of the claw 4343 and the magnetic member of the projection 4181 are attracted to each other, thereby locking the locking member 434, so that the sealing between the liquid filling cover 43 and the upper cover 41 is stronger. When the liquid filling cover 43 is in the unlocked position, the first connecting portion 422 is disengaged from the second connecting portion 421, the circumference of the inserting opening 4154 no longer abuts the claw 4343, so that the liquid filling cover 43 can be flipped. It can be understood that in other embodiments, the first connecting portion 422 and the second connecting portion 421 can adopt other shapes and connections, for example, the first connecting portion 422 and the second connecting portion 421 each include a claw 4181, the claw 4181 of the second connecting portion 421 has the same shape as that in this embodiment, but the first connecting portion 422 inverts its claw 4181 to engage with the claw 4181 of the second connecting portion 421.

The surface of the locking member 434 away from the cover body 433 is provided with a second connecting member 4345 disposed adjacent to the limiting groove 4155 of the stopping member 415. One end of the second connecting member 4345 away from the locking member 434 extends outwards to form a limiting portion 4346. The limiting portion 4346 cooperates with the limiting groove 4155 and can slide in the limiting groove 4155. A pivotal hole 4347 is formed in the side wall of the limiting portion 4346. In this embodiment, the liquid filling cover 43 further includes a rotating shaft 431, the rotating shaft 431 is mounted in the pivotal hole 4347 and is slidably fitted in the sliding grooves 4156. When the rotating shaft 431 is moved to one end of the sliding groove 4156 away from the inserting opening 4154, the liquid filling cover 43 can be flipped relative to the upper cover 41 around the rotating shaft 431 when turning over the liquid filling cover 43. It should be noted that when the rotating shaft 431 is stopped at any position in the sliding groove 4156, the resisting portion 4191 of the elastic member 419 is elastically resisted by the limiting portion 4346.

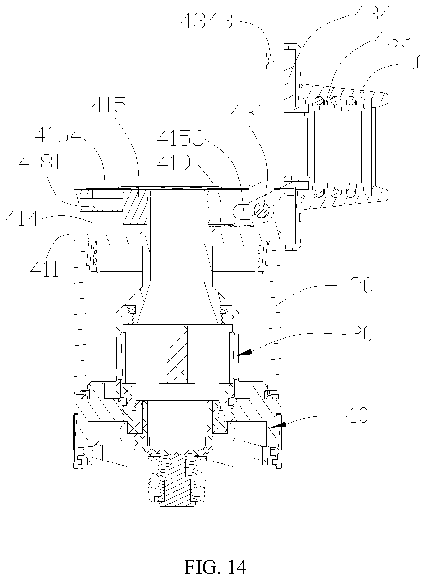

Referring to FIG. 13 and FIG. 14 simultaneously, when the user needs to fill liquid, the liquid filling cover 43 is firstly pushed towards the unlocked position, so that the locking member 434 moves under the driving of the liquid filling cover 43 to unlock the first connecting portion 422 and the second connecting portion 421, for example, the projection 4181 is disengaged from the latching groove 4344 of the claw 4343. When the claw 4343 is aligned with the inserting opening 4154, the rotating shaft 431 just moves to the end of the sliding groove 4156 away from the inserting opening 4154, and the liquid filling cover 43 is in the unlocked position. Then, the liquid filling cover 43 is flipped, so that the liquid filling hole 412 is exposed and opened, and the user can perform the liquid filling operation. Referring to FIG. 12, when the user finishes the liquid filling operation and needs to close the liquid filling hole 412, the liquid filling cover 43 is firstly flipped, so that the claw 4343 is received in the hollow chamber 4153 through the inserting opening 4154, and at this stage, the liquid filling cover 43 is in the unlocked position. Then, the liquid filling cover 43 is pushed toward the locked position, so that the latching groove 4344 and the projection 4181 are engaged with each other, and the circumference of the inserting opening 4154 abuts the claw 4343, thereby restricting the flipping of the locking member 43 relative to the upper cover 41, that is, the liquid filling cover 43 cannot be flipped relative to the upper cover 41. At this time, the liquid filling cover 43 is in the locked position, the first connecting portion 422 and the second connecting portion 421 are interlocked, the liquid filling cover 43 covers the upper cover 41, and the liquid filling hole 412 is closed, so that the user can inhale, and the liquid filling operation cannot be performed.

It should be noted that, in this embodiment, when the liquid filling cover 43 is in the locked position, due to the resistance of the elastic member 419 to the limiting portion 4346, and further due to the engagement of the latching groove 4344 and the projection 4181, the liquid filling cover 43 is always maintained in the locked position, and the situation that the liquid filling cover 43 slides to the unlocked position easily due to a small locking force is avoided. It can be understood that in other embodiments not shown, the presence of the elastic member 419 and the engagement of the latching groove 4344 and the projection 4181 can be retained only one, to also achieve the effect of preventing the liquid filling cover 43 from sliding easily.

In the electronic cigarette provided according to the second embodiment of the present invention, compared with the first embodiment, the position of the liquid filling hole 412 on the bottom cover 414 is unrestricted, which is convenient for production. By combining the pushing operation and the flipping operation, the liquid filling hole 412 can be opened, to completely expose the liquid filling hole 412 for liquid filling operation conveniently. Meanwhile, by providing the engagement structure of the projection 4181 and the latching groove 4344, the sealing between the liquid filling cover 43 and the upper cover 41 becomes more stably.

The above-mentioned embodiments merely represent several implementations of the present invention, and the descriptions thereof are more specific and detailed, but they shall not be understood as a limitation on the scope of the present invention. It should be noted that, for those of ordinary skill in the art, variations and improvements may be made without departing from the concept of the present invention, and all of which shall fall into the protection scope of the present invention. Therefore, the scope of protection of the present invention shall be subject to the appended claims.

## Claims

1. An upper cover assembly comprising an upper cover and a liquid filling cover movable relative to the upper cover between a locked position and an unlocked position, the upper cover being provided with a liquid filling hole; when the liquid filling cover is in the locked position relative to the upper cover, the liquid filling cover covers the upper cover and is unable to be flipped relative to the upper cover, and the liquid filling hole is closed; and when the liquid filling cover is moved, by an external force, from the locked position to the unlocked position relative to the upper cover, the liquid filling cover is able to be flipped and opened relative to the upper cover, and the liquid filling hole is exposed.

2. The upper cover assembly of claim **1**, wherein the upper cover is provided with a sliding groove for the liquid filling cover to move between the locked position and the unlocked position, the liquid filling cover is provided with a rotating shaft, the rotating shaft is movable along the sliding groove, the liquid filling cover is able to be flipped relative to the upper cover around the rotating shaft.

3. The upper cover assembly of claim **2**, wherein the upper cover comprises a bottom cover and a gland disposed on the bottom cover, the liquid filling hole is provided in the bottom cover, an edge of one side of the gland is recessed inwardly to form a notch having an opening at one end, the liquid filling hole is received in the notch and exposed through the notch.

4. The upper cover assembly of claim **3**, wherein the notch comprises a connecting wall located at a side of the liquid filling hole and two side walls connected to opposite ends of the connecting wall and extending in the same direction, the surface of the connecting wall near the bottom cover is recessed to form a recess, the surface of each of the two side walls near the bottom cover is recessed to form the sliding groove at the position near the connecting wall, the sliding groove is in communication with the notch and the recess; one end of the liquid filling cover is received and confined in the recess in the locked position; and when the end of the liquid filling cover is moved out of the recess along the sliding groove from the locked position to the unlocked position, the liquid filling cover is able to be flipped.

5. The upper cover assembly of claim **4**, wherein the liquid filling cover comprises a cover body, the cover body is able to close the liquid filling hole when the liquid filling cover is in the locked position, the rotating shaft is disposed at two opposite sides of one end of the cover body.

6. The upper cover assembly of claim **5**, wherein the liquid filling cover comprises a covering portion disposed on the other end of the cover body opposite to the rotating shaft, the covering portion extends to both sides of the cover body and has a width greater than the width of the notch in the circumferential direction of the gland, the bottom of the gland on both sides of the notch is provided with a locking groove in communication with the notch, the width of the locking groove is larger than the width of the notch in the circumferential direction of the gland; when the liquid filling cover is in the locked position, the covering portion is received in the locking groove and resists the groove wall of the locking groove; and when the liquid filling cover is in the unlocked position, the covering portion moves out of the locking groove, the liquid filling cover is able to be flipped relative to the upper cover.

7. The upper cover assembly of any one of claims **2** to **6**, wherein the upper cover is further provided with a smoke outlet, the smoke outlet and the liquid filling hole are sequentially arranged in the upper cover along the direction from the locked position to the unlocked position, the liquid filling cover is provided with a through hole, the through hole is in communication with the smoke outlet when the liquid filling cover is in the locked position relative to the upper cover.

8. The upper cover assembly of claim **1**, wherein the upper cover comprises a first connecting portion for restricting the liquid filling cover from being flipped when the liquid filling cover is in the locked position, and the liquid filling cover is provided with a second connecting portion for being locked or unlocked with the first connecting portion.

9. The upper cover assembly of claim **2**, wherein the upper cover comprises a bottom cover and a stopping member, the bottom cover is provided with an engaging groove, the stopping member is engaged in the engaging groove, two opposite side walls along the length direction of the stopping member are provided with the sliding groove.

10. The upper cover assembly of claim **9**, wherein the liquid filling cover comprises a locking member, the locking member closes the liquid filling hole when the liquid filling cover is in the locked position, the rotating shaft is provided on the locking member, the rotating shaft is movable along the sliding groove to be locked with the stopping member.

11. The upper cover assembly of claim **10**, wherein the surface of the stopping member away from the liquid filling cover is defined with a clamping groove, one end of the stopping member opposite to the clamping groove is provided with a limiting groove, one end of the locking member is provided with a claw slidably received in the clamping groove, the other end of the locking member opposite to the claw is provided with a limiting portion slidably received in the limiting groove, the rotating shaft is provided on the limiting portion.

12. The upper cover assembly of claim **11**, wherein a surface of the stopping member facing the liquid filling cover is provided with an inserting opening in communication with the clamping groove, the claw is movable in the clamping groove after passing through the inserting opening, the claw is provided with a latching groove, a projection corresponding to the latching groove is provided in the clamping groove; when the liquid filling cover is in the locked position, the projection is snapped in the latching groove, the circumference of the inserting opening abuts against the claw.

13. The upper cover assembly of claim **8**, wherein the first connecting portion and the second connecting portion are each provided with a magnetic member; when the liquid filling cover is in the locked position, the first connecting portion is magnetically connected to the second connecting portion.

14. The upper cover assembly of claim **11**, wherein the upper cover further comprises an elastic member, the elastic member comprises a clamping portion clamped between the bottom cover and the stopping member and a resisting portion connected to the clamping portion, the resisting portion is connected to one end of the clamping portion away from the inserting opening, one end of the resisting portion away from the clamping portion is inclined in a direction away from the bottom wall of the engaging groove; when the liquid filling cover moves between the locked position and the unlocked position, the resisting portion always abuts against the limiting portion.

15. The upper cover assembly of claim **10**, wherein the upper cover further comprises an upper cover casing sleeved on the bottom cover, the liquid filling cover further comprises a cover body connected to one side of the locking member away from the upper cover, the upper cover casing is provided with a smoke outlet, the cover body is provided with a through hole, the through hole is in communication with the smoke outlet when the liquid filling cover is in the locked position relative to the upper cover.

16. An atomizing device comprising the upper cover assembly according to any one of claims **1** to **15**.

17. The atomizing device of claim **16**, wherein the atomizing device further comprises a base assembly, a liquid storage tube and an atomizing assembly, the liquid storage tube is sleeved on the base assembly, a liquid storage chamber is surrounded cooperatively by the liquid storage tube and the base assembly, the atomizing assembly is received in the liquid storage chamber, the upper cover assembly is disposed at one end of the liquid storage tube opposite to the base assembly and is in communication with the liquid storage chamber through the liquid filling hole.

18. An electronic cigarette comprising the atomizing device according to claim **16** or **17**.
